# EUROPEAN PATENT SPECIFICATION

(11) **EP 3 744 234 B1**
(45) Date of publication and mention of the grant of the patent: **10.08.2022**
(21) Application number: 20156876.3
(22) Date of filing: 12.02.2020
(51) Int. Cl.: A61B 5/00, A61B 5/03, A61B 5/316, A61B 5/389

(54) **A DEVICE AND A METHOD FOR NON-INVASIVE EVALUATION OF FUNCTIONAL STABILITY OF THE TRUNK**
VORRICHTUNG UND VERFAHREN ZUR NICHT-INVASIVEN BEWERTUNG DER FUNKTIONELLEN STABILITÄT DES RUMPFES
DISPOSITIF ET PROCÉDÉ D'ÉVALUATION NON INVASIVE DE LA STABILITÉ FONCTIONNELLE DU TRONC

(43) Date of publication of application: 02.12.2020
(73) Proprietor: Univerza na Primorskem, 6000 Koper (SI)
(72) Inventor: Sarabon, Nejc, 4000 Kranj (SI); Markovic, Goran, 10000 Zagreb (HR)
(74) Representative: Patentni Biro AF d.o.o.

(56) References cited:
- US-A- 5 052 406
- US-A1- 2005 283 056
- US-A1- 2012 083 798
- VOGLAR MATEJ ET AL: "Reflex delays of the trunk muscles in response to postural perturbations: A reliability study", JOURNAL OF BIOMECHANICS, PERGAMON PRESS, NEW YORK, NY, US, vol. 47, no. 11, 9 June 2014 (2014-06-09), pages 2807-2812, XP029015291, ISSN: 0021-9290, DOI: 10.1016/J.JBIOMECH.2014.05.024

## Description

### Field of the invention

The present invention belongs to the field of medical devices, more precisely to the field of measuring devices. The object of the invention is a device for non-invasive evaluation of the functional stability of the trunk comprising an external perturbation module, which allows for a fast, easy and accurate analysis of the functional stability of the trunk. The invention also relates to a method for evaluation of trunk stability using said device.

### Background of the invention and the technical problem

The torso or trunk is an anatomical term for the central part of human body, from which the neck and limbs extend. The torso includes the thoracic segment of the trunk, the abdominal segment of the trunk and the pelvic girdle segment. The torso houses majority of the vital organs, such as heart and lungs, organs involved in digestion and reproductive organs. Said organs are protected by a multitude of bones and muscles, which ensure proper stability and resistance to external (tripping, slipping, catching a load, etc.) or internal (lifting a load, throwing, etc.) mechanical perturbations. Generally, the body core is stabilized by actively controlling physical stability and tension against gravity through interaction between various somatosensors, sensory nerves, conduction and processing central nervous system centres, motor nerves as well as lumbar and abdominal muscles. Core stability refers to a person's ability to stabilize his/her trunk, i.e. to control the position and movement of the trunk. Thus, greater core stability means greater level of control over the position, stiffness, and movement of this area of the body. The correlation between core strength and spinal health is widely accepted and intra-abdominal pressure is involved in increasing spine stability. Spinal stability in general and lower back specifically are to a huge extent (-90%) assured by so called active stability, which comes from muscle action in either anticipatory/preprogramed or reactive/reflexive types of action.

Decreased stability of the trunk may result in low back pain, wherein the latter is classified as:
- acute if the duration of pain is less than 6 weeks,
- subacute if the duration is between 6 weeks and 12 weeks, and
- chronic if the duration is over 12 weeks.

Most low back pain episodes last for 2 to 3 months but may repeat and become chronic. About 80% of all low back pain has no known mechanism, i.e. structural pathology, thus being classified as non-specific idiopathic low back pain. Based on current scientific evidence, this comes from the fact that standard medical diagnostic procedures put focus on imaging techniques and structural pathology. However, huge body of evidence from the past 20-30 years indicate the importance of testing functional aspect in order to make diagnosis more complete and a better support to therapy guidance. Sensory-motor and neuro-muscular functions related to trunk are in this context of the central interest.

It is important to evaluate trunk stability in diagnosis as well as treatment and therapy of chronic low back pain and also other conditions. This may reflect in impaired balance, particularly in elderly people, and/or in lower overall movement quality (certain biomechanical patterns leading to injuries of lower and upper extremities). Usually the trunk stability functions are evaluated with electrodes measuring electrical activity of muscles and/or with invasive sensors for measuring pressure inside the abdomen, wherein the said sensors are inserted to the abdominal cavity through the pharynx or anus or urinary tract. All of these are invasive techniques inconvenient for wider use.

The general aim of the present invention is thus development of a device for non-invasive evaluation of the functional stability of the trunk (NEFST) that would combine measurement of several parameters involved in trunk stability. In the field of measuring systems, no such devices have yet been developed. Hence, the technical problem, which is solved by the present invention, is a constructional design of a device that will enable evaluation of trunk stability upon mechanical perturbation of trunk posture in a fast, reliable and non-invasive manner, wherein the device should be based on an inflatable cuff placed around the subject's torso.

### State of the art

In medicine, the use of inflatable cuffs is otherwise widely practised in the field of blood pressure monitoring, with the cuff positioned at different locations on the limbs or fingers. Inflatable cuffs, placed around the lower back, are used for other purposes than that envisaged by the present invention, namely for monitoring or stimulation during childbirth or for abdominal decompression (e.g., US 5893368, US 5174281 and DE 3729760).

An inflatable cuff placed around the subject's torso has been disclosed in patent EP 0975260 B1, however the said cuff is intended for fast fixation of different electrodes placed on skin. Similar has also been disclosed by Sadleir et al. (2000) in the article "Inflatable belt for the application of electrode arrays" published in Rev. Sci. Instrum. 71, 530. In the article the belt comprises fields with electrodes and the belt can be inflated by segments in order to press individual electrodes against the skin with the aim of improving the "signal/noise" ratio.

A method for non-invasive detection or monitoring of intra-abdominal pressure was presented in CN 100346742, where a completely different technical solution was used that does not allow for a comprehensive evaluation during the performance of various activities. This document discloses a measuring probe, which is pressed by the measurer using the hand against the subject's abdomen at a single point.

Similar as the latter, in US 6,185,451 B1 the pressure and electrical activity of the muscles are acquired while lying prone with the aim of feedback for activation of abdominal muscles to search for deep muscle strengthening exercises. This document describes a method and an apparatus for assessing the function of deep joint stabilizing muscles, wherein the apparatus includes an EMG unit, an ultrasound unit, a pressure biofeedback unit and a vitalograph, in combination with a computer programmed to analyse data from them and given an indication of function. The pressure biofeedback unit has a pressure pad, which is placed under the subject's abdomen when lying prone, which differs significantly from the present invention. In this design, the device according to US 6,185,451 B1 cannot be used in combination with the mechanical perturbation module according to the invention.

Document DE 4205790 discloses a feedback-based system for active posture correction; however, the sensory and mechanical solutions differ from the present invention.

Voglar and Sarabon describe use of surface electromyography for evaluation of trunk muscle activity, while intraabdominal pressure has not been determined.

Patent application US2012083798 discloses an equipped modular inflatable belt as aid to labour and delivery comprising a supporting band and at least one inflatable chamber applied to said band provided with ducts for connection to a supply of an inflating fluid, said belt comprising an electropneumatic apparatus applied to the band. The inflatable belt that is able to exert different pressures in distinct areas of application of the belt, according to the clinical requirements at a given moment, and simultaneously to monitor the bioelectrical parameters of the mother and foetus. This device is not arranged to measure intraabdominal pressure related to trunk stability.

Patent application US2005283056 describes an apparatus for controlling the childbirth labour comprising: an electromyographic unit with sensors associated therewith for detecting the electrical signals of a parturient's uterus and means for analysing and processing said electrical signals, and a pneumatic belt to be fixed around the parturient's abdomen and having sensors associated therewith to detect the variation of the internal pressure due to uterine contractions, and means for inflating said belt to provide a thrust as an aid for the expulsion of the fetus, characterized in that a device is provided, associated with said sensors to control the activation of said means for the inflation of the pneumatic belt. This solution differs from the present invention as it does not allow determination of trunk stability. Document US5052406 describes methods and devices for evaluating among the trunk and limbs of the body the distribution of impairments of two types of abilities necessary for posture and equilibrium control: (1) ability to receive and correctly interpret somatosensory orientation and movement information derived from those body and limb parts in contact with supporting surfaces and (2) ability to coordinate the muscular contractions in those body and limb parts in contact with a supporting surface to execute functionally effective postural movements. Said devices differ from the present invention.

### Description of the solution of the technical problem

The technical problem has been solved with the invention as defined in the independent claim, wherein preferred embodiments of the device are defined in the dependent claims.

The present invention is a device for non-invasive evaluation of the functional stability of the trunk comprising a mechanical perturbation module, a sensory module synchronized with the mechanical perturbation module and an analysis module connected with the sensory module. The essence of the invention is in that the said device comprises:
- an inflatable cuff to be placed around a subject's waist,
- at least one electrode mounted on the said cuff for measuring muscle activity of the lumbar and abdominal muscles,
- at least one pressure sensor for measuring pressure inside the cuff corresponding to subject's intra-abdominal pressure, and
- an analysis module, and
a central receiver unit for receiving and transmitting signals from the said electrodes and said pressure sensor to the analysis module; wherein the electrodes and the sensor measure pressure and muscle activity upon controlled application of external perturbations of the subject's trunk caused by a perturbation module. The device preferably has one pair of said electrodes, more preferably more, such as four (two pairs) to obtain a wider overview of muscle activity. On the other hand, one pressure sensor suffices for reliable results, however one could use more pressure sensors distributed in different compartments of the cuff and then use values from each compartment separately (torso region specific) or calculate the average pressure.

The central receiver unit is connected to the input/output synchronization unit, which is connected to a computer and the perturbation module. Throughout the entire measurement process, signals from the pressure sensor and the electrodes are synchronized with signals from the mechanical perturbation module. The sensory module is synchronized with the mechanical perturbation module in order to ensure synchronization of the perturbation with measurements performed with electrodes and pressure sensor(s). The whole analysis has to be done with regards to the trigger-signal moment, as besides amplitudes of muscle activity and pressure also time parameters are important. The signals are then transmitted to the computer, which captures stores and subsequently processes the signals. The device may thus include a suitable computer program for automatic analysis of obtained data in order to evaluate subject's trunk stability.

The cuff is designed in any suitable way, as long as it has at least one closed inflatable chamber. It can be designed as a flexible belt or as a cuff with outer harder housing (shell) made of plastics or composite materials and an inner inflatable chamber made of flexible material. The latter could be made as one piece or as two-piece cuff, wherein at least one hinge connects both parts so that it can be placed around a person's waist as tongs. The inflatable cuff is preferably a substantially elongated closed chamber with an opening, into which a device for delivering into or removing air from the device is installed. This device may be any known in the field and can be operated manually or automatically. The cuff is also equipped with suitable attachment means, which allow the cuff to be placed around a subject's torso and reliably fastened. This is achieved by, for example, tapes with hooks and loops, clamps or any other attachment means known in the field. The length of the cuff is arbitrary, wherein the attachment means allow suitable variations in the length. The width of the cuff may be from ten to thirty centimetres, preferably about fifteen centimetres. The cuff is placed so that it covers at least the area between the upper line of the pelvis (crista iliaca) and lowest line of the rib cage. The inflatable cuff is preferably made of two or more layers and has one or more compartments.

The device measures at least two essential parameters upon an external perturbation, namely muscle activity of the lumbar and abdominal muscles and intra-abdominal pressure. The latter is measured indirectly via the pressure sensor for measuring the pressure inside the inflatable cuff. Statistical validity of this approach to indirect abdominal pressure measurement was confirmed in our preliminary studies. This finding was in line with our hypothesis based on the following facts: (i) abdominal cavity is limited by muscle groups in all directions - abdominal diaphragm muscle superiorly, pelvic flor muscles inferiorly, abdominal wall and back muscles at perimeter; (ii) relative compressing or decompressing of the volume in abdominal cavity comes from activation or relaxation of these muscle groups, respectively; (iii) as muscles are not mechanically rigid structures, any increase or decrease of intra-abdominal pressure mirrors in geometrical deformation of the external walls of the abdominal cavity; (iv) the abdominal wall and back muscles at perimeter of the abdominal cavity are strongly indicative of this intra-abdominal pressure change regarding the tension change at the perimeter. The sensor may be placed anywhere, as the pressure inside the cuff is approximately uniform. In case the cuff has more than one compartment, each of the compartments has at least one pressure sensor. In the preferred embodiment, the sensor is placed near the housing for housing electronics. Said sensor may be conductive rubber, conductive textile, self-adhesive sensor, metal sensors of different types (Ag-AgCI, Au, Cu or other) and any other suitable pressure sensor known in the art. Obtaining an indicative value of intra-abdominal pressure is important for the evaluation of trunk stability, as higher intra-abdominal pressure has been linked with higher spine stability.

The muscle activity is followed with any suitable electrode able to measure muscle activity, such as EMG electrodes for measuring electrical activity of muscles, especially textile EMG electrodes and conductive rubber electrodes. The number of said electrodes is at least one, preferably two or more, most preferably four. Four electrodes enable most reliable measurement, wherein more electrodes are possible but not required from the point of device complication and price. The electrodes are preferably movably mounted and/or attached in/on a suitable holder, so that they can be relocated anywhere on the belt in order to ensure correct position with regards to the subject's body measurements and shape. The electrodes are preferably placed on at least one muscle selected in the group consisting of:
- m. obliques abdominis externus,
- m. obliques abdominis internus,
- m. rectus abdominis,
- m. errector spinae,
- m. multifidus;
preferably onto more of said muscles or any additional abdominal/lumbar muscle involved in core stability. Each electrode is connected via thin conductive wires to the central receiver unit.

Additionally, the device may comprise any further sensor or electrode or any other device for following other aspects important in trunk stability. For example, centre of gravity could be measured with a suitable plate for measuring forces of the human body onto the plate. Breathing could also be followed, but it is usually contributing to the intra-abdominal pressure, which is already measured.

The central receiver unit and the input/output synchronization unit comprise of (a) the amplifiers which amplify the force sensor, accelerometer, pressure and EMG signals; (b) analogue-to-digital transmission unit; (c) digital input/output unit for the control of the mechanical perturbation triggering; (d) the data acquisition unit enabling the signals to be read by a computer in a time synchronised way; and (e) custom build computer software for storage, visualisation, processing/quantification, and reporting of the acquired signals.

The mechanical perturbation module employs destabilization manoeuvres, i.e., an external perturbation, wherein a suitable mechanism releases a pre-determined tension.

The external perturbation module ensures suitable perturbation simulating sudden movements such as preventing a fall, catching a load, lifting a load, etc. While the mechanical perturbation module may be designed in any suitable way, it is preferably designed as a holder or a pole to be held in subject's hands connected to a restraining/pulling element with a rope or a wire, which can be pulled in or out of the restraining/pulling element. Such perturbation module thus allows two modes of operation:
- the restraining/pulling element functions as a restraining device, which applies certain force which prevents pulling of the rope or the wire upwards; the subject holding the pole aims to pull and hold the pole horizontally to the ground at the shoulder level, consequently activating core muscles; and
- the restraining/pulling element functions as a pulling device, which is pulling the rope or the wire into the element, wherein the subject holding the pole aims to maintain the pole horizontally to the ground at the shoulder level, which also results in activating core muscles.

The restraining/pulling element can be constructed in any suitable way obvious to a skilled person, which allows execution of above-described operation modes. Said restraining force or the pulling force applied to the rope may be triggered externally via manual or automatic control in a certain time point, wherein triggering may be repeated multiple times with any time gap. This means that the subject is holding the pole and in an unexpected time point restraining or pulling force is applied. This accurately mimics unexpected sudden movements mentioned above. The force may be varied between individual repeats, wherein they are usually controlled with pretensions in the module, which can be in the range from 10 to 200 N.

Alternatively, the perturbation module may also be simplified to allow examination after the above-mentioned inner perturbations, so that for example an accelerometer is provided for attachment onto subject's extremity, preferably wrist, the said accelerometer measuring the change in the velocity of arm movement in order to determine intended body movements. The tested person may also hold weights for example up to 5 kg in order to cause more significant perturbations.

The analysis module comprises the input/output synchronization unit, which is connected to the computer and the perturbation module. Throughout the entire measurement process, signals from the pressure sensor and the electrodes are synchronized with signals from the mechanical perturbation module. The signals are processed by the computer in order to obtain at least some of the following data:
- optionally muscle activity before and after the test;
- muscle activity before each perturbation;
- muscle activity during each perturbation;
- muscle activity after each perturbation;
- optionally intra-abdominal pressure before and after the test;
- intra-abdominal pressure before each perturbation;
- intra-abdominal pressure during each perturbation;
- intra-abdominal pressure after each perturbation;
- duration of each perturbation; and
- force applied on the pole during each perturbation.

The device may thus include a suitable computer program for automatic analysis of obtained data in order to evaluate subject's trunk stability through time and amplitude parameters of the EMG signals relative to the mechanical perturbation event(s). The said computer program preferably performs the following tasks: data acquisition and storage, signal processing including filtering and normalisation, trigger or start event automatic recognition, calculation of outcome measures in domain of time and amplitude.

The method for evaluation of trunk stability using the above-described device according to the invention comprises at least the following steps performed in the said sequence:
a) placing the inflatable cuff with at least one electrode and at least one pressure sensor around a subject's waist,
b) establishing contact between the subject and the perturbation module,
c) triggering at least one perturbation with the perturbation module,
d) measuring muscle activity and pressure inside the cuff,
e) receiving the signals from the sensor and the electrode to the central receiver,
f) transmitting signals to the input/output synchronization unit, which is connected to a computer and the perturbation module.
g) optionally processing and storing the signals in the computer, and
h) final evaluating of the subject's trunk stability based on obtained data in steps d) and optionally g).

Throughout the entire method, signals from the pressure sensor and the electrodes are synchronized with signals from the mechanical perturbation module. Preferably the measurements in step d) are also performed prior and after the perturbation. Additionally, measurements can be performed in the start and the end of testing. Perturbations are preferably applied more than once in order to obtain more accurate data, the preferred number of perturbations is 5 to 20, wherein they can be randomly distributed in a certain time period and can have different forces. The steps g) and h) may be performed automatically by the above-discussed computer program for automatic analysis of obtained data in order to evaluate subject's trunk stability.

The present invention is a neurophysiological and biomechanical measuring device, which can be used to accurately and reliably evaluate the functional stability of the trunk in healthy people as well as in patients within the disciplines of medicine and kinesiology. Use of the device and the method is thus primarily in kinesiology and medicine, especially when there is a need to assess and evaluate various deficits related to injury, surgery, neurological diseases, aging, etc. or effects of exercise such as physiotherapy, sports, research, sport medicine, injury prevention, etc.

However, uses related to the diagnosis of one of the above-mentioned conditions are not covered by the present invention.

During development and testing of the present invention it has been determined that the belt itself may be used also on its own without the mechanical perturbation module and analysis module, for example for measuring intra-abdominal pressure during controlled abdominal muscle contractions, for learning controlled increase of intra-abdominal pressure during load lifting, etc., however, the mechanical perturbation module represents the main feature of the present invention.

The device for non-invasive evaluation of the functional stability of the trunk will be described in more detail based on exemplary embodiments and figures, which show:
- Figure 1: Interior of the inflatable cuff according to a possible embodiment
- Figure 2: Exterior of the cuff according to the possible embodiment shown in Figure 1
- Figure 3: Cross-section of the cuff according to the possible embodiment shown in figures 1 and 2
- Figure 4: A possible embodiment of the mechanical perturbation module
- Figure 5: Exemplary use of the device according to the invention
- Figure 6a: Results of intra-abdominal pressure measurements in case of lifting weight forwards obtained with the device according to the invention (red) and a known nasopharyngeal probe (green)
- Figure 6b: Results of intra-abdominal pressure measurements in case of single-leg landing obtained with the device according to the invention (blue) and a known nasopharyngeal probe (red)

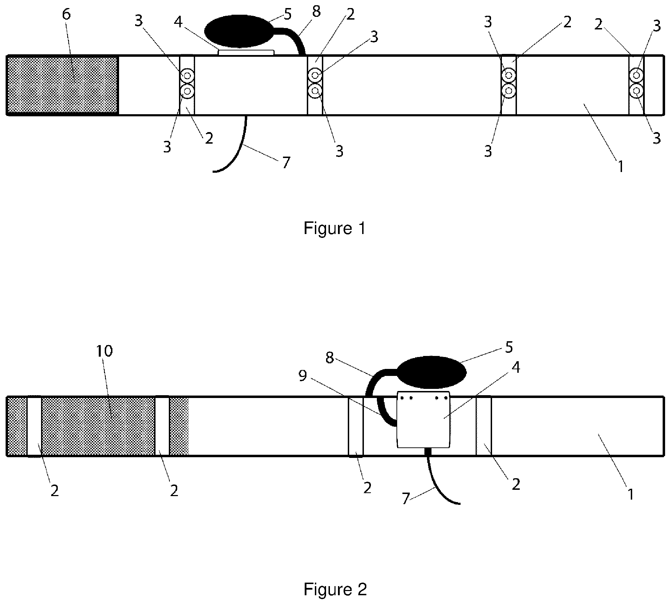

Figures 1 and 2 show the interior and the exterior of the inflatable cuff (1), respectively. The cuff is made of a material that does not change its form when inflated. Suitable materials are linen, carbon fibre fabric and Kevlar fabric (polyparaphenylene terephthalamide). The inflatable cuff (1) is made of two or more layers and has one or more compartments. Four electrode holders (2) are positioned on the cuff and they can be moved longitudinally along the inflatable cuff (1). The electrode holder (2) is made of textile; it is wrapped around the inflatable cuff (1) and is long enough to be moved along the inflated cuff (1). On the inside of the inflatable cuff (1) there is one pair of EMG electrodes (3) mounted on each electrode holder (2). Textile EMG electrodes are preferred, but any other type can be used as well. Each pair of EMG electrodes (3) is connected via thin conductive wires to the central receiver unit (4) where the input unit for EMG signals is located.

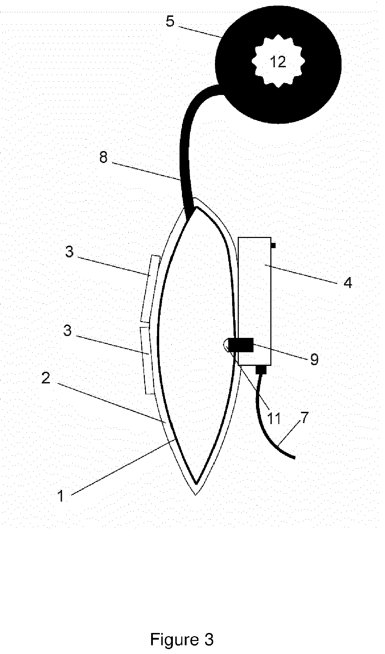

The inflatable cuff (1) is connected via a connecting tube (8) to a hand pump (5). The inflatable cuff is connected via a connecting tube (9) to a pressure sensor in the central receiver unit (4). The central receiver unit (4) consists of a pressure sensor, electronics to capture the EMG signals and a card for conversion of analogue signals into digital signals. The central receiver unit (4) is connected to the input/output synchronization unit (15) via a connecting cable (7). Figure 3 shows a cross-section through the cuff (1).

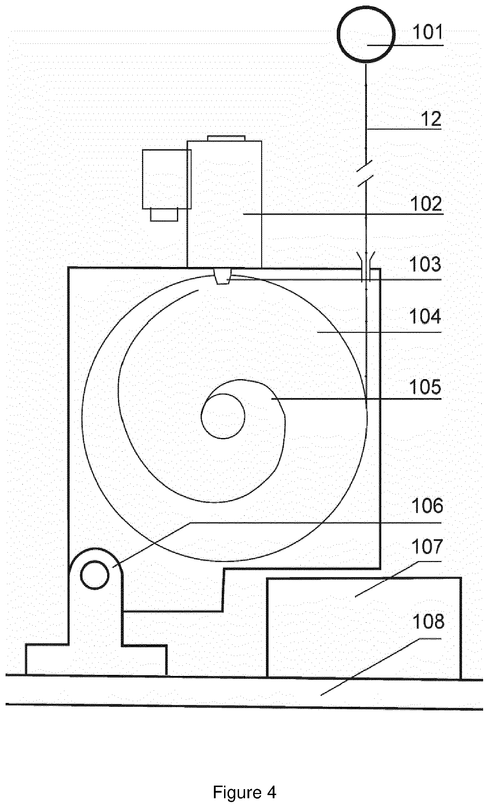

Figure 4 shows a possible perturbation module (100) for causing external perturbations, wherein the module (100) comprises: a holder or a pole (101) to be held in subject's hands connected with a rope (12) to a housing (104) of the module with a retaining element (105), which is preferably a spiral spring. The rope (12) can be pulled in or out of the housing (104), depending on the electro-mechanical mechanism to load or unload the wire, which is preferably a magnetic actuator (103) with a closure. The housing (104) is mounted onto a bottom or a plate (108) with a suitable mounting element (106). The plate (108) is also provided with a force sensor (107) for measuring the force exerted by the tested subject when pulling or restraining the rope (12) and the pole (101).

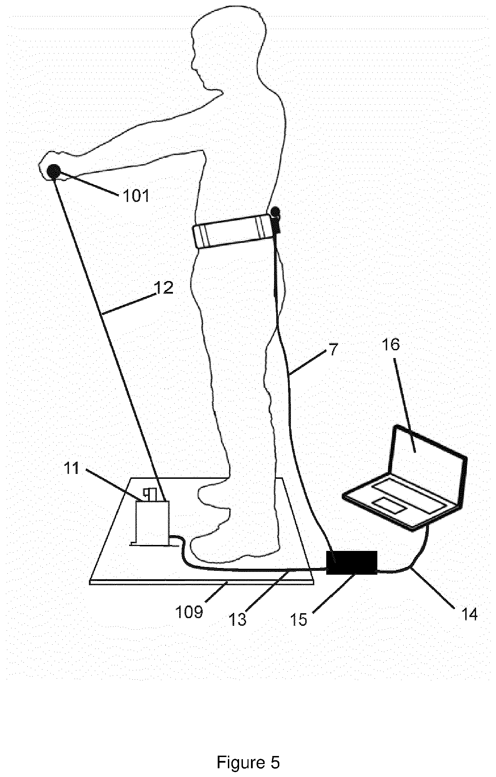

Exemplary use of the device according to the invention is shown in figure 5. When placed around the waist, the inflatable cuff is fastened by a hook and loop fastener, strips of which are positioned on the inside of one end (6) and on the outside of the other end of the cuff (10). Both hook and loop strips (6, 10) are long enough to be attached onto each other when the inflatable cuff (1) is placed around the waist of an adult or child. The tested subject stands on the plate (108) placed on floor or suitable stand. The perturbation module (11) has a wire or a rope (12) with a holder (101) adjustable to fit any person's height. The wire or the rope (12) is wound inside the housing of the perturbation module and is connected to the electro-mechanical mechanism to load or unload the wire. Perturbation module (11) is connected to the input/output synchronization unit (15) via a connecting cable (13). The input/output synchronization unit (15) is connected to the PC (16) via a USB cable (14).

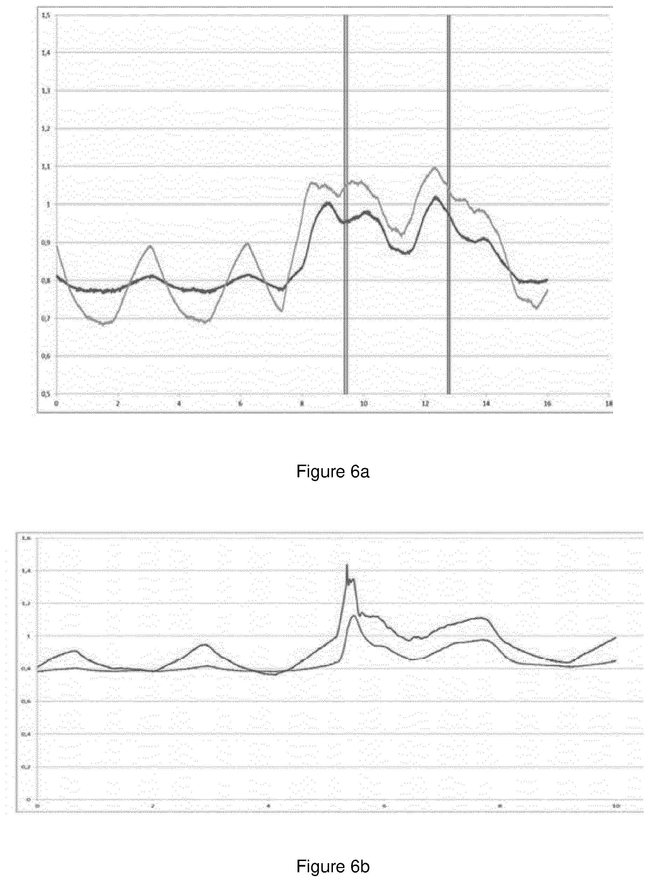

Figure 6a and 6b present preliminary results obtained using the inflatable cuff with embedded pressure sensor as described above, while the subject was performing two different functional tasks - load lifting (fig. 6a) and single-leg landing (Fig. 6b). At the same time correlation of said results with measurements using a nasopharyngeal probe are shown. In both figures, the x axis is time (sec) and the y axis presents intra-abdominal pressure (Pa). The cross-correlation of results obtained with the cuff and known nasopharyngeal probe in both examples is higher than 0.95, which implies high reliability of results obtained with the device according to the invention confirming its usefulness in future non-invasive evaluation of functional stability of the trunk.

## Claims

1. A device for non-invasive evaluation of the functional stability of the trunk comprising:
- a sensory module, wherein the sensory module comprises:
∘ an inflatable cuff (1) to be placed around a subject's waist,
∘ at least one electrode (3) mounted on the said cuff (1) for measuring muscle activity of the lumbar and abdominal muscles,
∘ at least one pressure sensor for measuring pressure inside the cuff (1) corresponding to the subject's intra-abdominal pressure,
- an analysis module, and a central receiver unit (4) for receiving and transmitting signals from said electrodes (2) and said pressure sensor to the analysis module, **characterized in that**
- the device further comprises a mechanical perturbation module (100),
- wherein the sensory module is synchronized with the mechanical perturbation module (100), and
- wherein the electrodes (3) and the sensor are configured to measure muscle activity and pressure, respectively, upon controlled application of perturbations of the subject's trunk caused by the perturbation module.

2. The device according to claim 1, **characterized in that** the mechanical perturbation module employs destabilization manoeuvres, namely:
- an external perturbation, wherein a suitable mechanism is configured to release a pre-determined tension.

3. The device according to any of the preceding claims, **characterized in that**
- the central receiver unit (4) is connected to the input/output synchronization unit (15), which is connected to a computer and the perturbation module (100), wherein throughout the entire measurement process, signals from the pressure sensor and the electrodes (3) are synchronized with signals from the mechanical perturbation module (100), and
- wherein the signals are then transmitted to the computer, which captures stores and subsequently processes the signals, which may further include a suitable computer program for automatic analysis of obtained data in order to evaluate subject's trunk stability.

4. The device according to any of the preceding claims, **characterized in that** the device further comprises any sensor or electrode or any other device for following other aspects important in trunk stability, such as a force plate for following centre of pressure.

5. The device according to any of the preceding claims, **characterized in that**
- the cuff (1) has at least one closed inflatable chamber, wherein the preferred design is as a flexible belt or as a cuff with outer harder housing made of plastics or composite materials and an inner inflatable chamber made of flexible material;
- **in that** the cuff (1) is provided with suitable attachment means (6), which allow the cuff (1) to be placed around a subject's torso and reliably fastened; and
- **in that** the width of the cuff (1) may be from five to thirty centimetres, preferably about fifteen centimetres, so that it covers at least the area between the upper line of the pelvis (crista iliaca) and lowest line of the rib cage.

6. The device according to the preceding claim, **characterized in that** the inflatable chamber is a substantially elongated closed chamber with an opening, into which a device for delivering into or removing air from the chamber is installed, wherein the chamber is made from linen, carbon fibre fabric or Kevlar fabric (polyparaphenylene terephthalamide).

7. The device according to any of the preceding claims, **characterized in that**:
- at least one pressure sensor is placed anywhere inside the cuff (1), or in case the cuff (1) has more than one compartment, each of the compartments has at least one pressure sensor, wherein the said sensor may be conductive rubber, conductive textile, self-adhesive sensor, metal sensors of different types such as Ag-AgCI, Au, Cu; and
- that muscle activity is followed with any suitable electrode (3) able to measure muscle activity, such as EMG electrodes for measuring electrical activity of muscles, especially textile EMG electrodes and conductive rubber electrodes, wherein the number of said electrodes is at least one, preferably two or more, most preferably four.

8. The device according to the preceding claim, **characterized in that** the electrodes (3) are movably mounted and/or attached in/on a suitable holder (2), so that they can be relocated anywhere on the belt in order to ensure correct position with regards to the subject's body measurements and shape, wherein the electrodes are preferably placed on at least one muscle selected in the group consisting of:
- m. obliques abdominis externus,
- m. obliques abdominis internus,
- m. rectus abdominis,
- m. errector spinae,
- m. multifidus;
preferably onto more of said muscles or any additional abdominal/lumbar muscle involved in core stability.

9. The device according to any of the preceding claims, **characterized in that** the perturbation module (100) is designed as a holder or a pole (101) to be held in subject's hands connected to a restraining/pulling element with a rope or a wire (12), which can be pulled in or out of the restraining/pulling element (105); wherein the perturbation module thus allows two modes of operation:
- the restraining/pulling element functions as a restraining device, which is configured to apply certain force which prevents pulling of the rope or the wire (12) upwards; the subject holding the pole aims to pull and hold the pole (101) horizontally to the ground at the shoulder level, consequently activating core muscles; and
- the restraining/pulling element functions as a pulling device, which is configured to pull the rope or the wire (12) into the element (105), wherein the subject holding the pole aims to maintain the pole (101) horizontally to the ground at the shoulder level, which also results in activating core muscles.

10. The device according to any claim from 1 to 8, **characterized in that** the perturbation module (100) comprises an accelerometer for measuring the change in the velocity of arm movement.

11. The device according to any of the preceding claims, **characterized in that** the analysis module comprises the input/output synchronization unit (15), which is connected to the computer and the perturbation module, wherein throughout the entire measurement process, signals from the pressure sensor and the electrodes (3) are synchronized with signals from the mechanical perturbation module (100), and wherein the signals are processed by the computer in order to obtain at least some of the following data - in time or amplitude domain:
- optionally muscle activity before and after the test;
- muscle activity before each perturbation;
- muscle activity during each perturbation;
- muscle activity after each perturbation;
- optionally intra-abdominal pressure before and after the test;
- intra-abdominal pressure before each perturbation;
- intra-abdominal pressure during each perturbation;
- intra-abdominal pressure after each perturbation;
- duration of each perturbation; and
- force applied on the pole during each perturbation.

12. The device according to any of the preceding claims, **characterized in that** the central receiver unit (4) and the input/output synchronization unit (15) comprise (a) the amplifiers which are configured to amplify the force sensor, accelerometer, pressure and EMG signals; (b) analogue-to-digital transmission unit; (c) digital input/output unit for the control of the mechanical perturbation triggering; (d) a data acquisition unit for enabling the signals to be read by a computer in a time synchronised way; and (e) custom build computer software for storage, visualisation, processing/quantification, and reporting of the acquired signals.

13. A method for evaluation of trunk stability using the above-described device according to any of the preceding claims, **characterized in that** the method comprises at least the following steps:
a) placing the inflatable cuff (1) with at least one electrode (3) and at least one pressure sensor around a subject's waist,
b) establishing contact between the subject and the perturbation module (100),
c) triggering at least one perturbation with the perturbation module (100),
d) measuring muscle activity and pressure inside the cuff (1),
e) receiving the signals from the sensor and the electrode (3) to the central receiver (4),
f) transmitting signals to the input/output synchronization unit (15), which is connected to a computer and the perturbation module (100),
g) optionally processing and storing the signals in the computer, and
h) final evaluating of the subject's trunk stability based on obtained data in steps d) and optionally g); all in time or amplitude domain.

14. The method according to the preceding claim, **characterized in that** perturbations are at least once, preferably applied more than once in order to obtain more accurate data, wherein the preferred number of perturbations is 5 to 20, which can be randomly distributed in a certain time period and can have different forces.

15. The method according to any claim from 13 to 14, **characterized in that** inner perturbations are enhanced by using weights, for example up to 5 kg.

## Patentansprüche

1. Vorrichtung und Verfahren zur nicht-invasiven Bewertung der funktionellen Stabilität des Rumpfes, umfassend
- ein Sensormodul, wobei das Sensormodul umfasst:
∘ eine aufblasbare Manschette (1), die um die Taille einer Person gelegt wird,
∘ mindestens eine an der Manschette (1) angebrachte Elektrode (3) zur Messung der Muskelaktivität der Lenden- und Bauchmuskulatur,
∘ mindestens einen Drucksensor zur Messung des Drucks im Inneren der Manschette (1), der dem intraabdominalen Druck der Person entspricht,
- ein Analysemodul, eine zentrale Empfangseinheit (4) zum Empfangen und Übertragen von Signalen von den Elektroden (3) und dem Drucksensor an das Analysemodul,
**dadurch gekennzeichnet, dass**
- die Vorrichtung ferner ein mechanisches Perturbationsmodul (100) umfasst,
- wobei das Sensormodul mit dem mechanischen Perturbationsmodul (100) synchronisiert ist, und
- wobei die Elektroden (3) und der Sensor konfiguriert sind, um die Muskelaktivität bzw. den Druck bei kontrollierter Anwendung von durch das Perturbationsmodul verursachten Perturbationen des Rumpfes einer Person messen.

2. Vorrichtung nach Anspruch 1, **dadurch gekennzeichnet, dass** das mechanische Perturbationsmodul Destabilisierungsmanöver anwendet, nämlich:
- eine externe Perturbation, wobei ein geeigneter Mechanismus so konfiguriert ist, um eine vorbestimmte Spannung zu lösen.

3. Vorrichtung nach einem der vorstehenden Ansprüche, **dadurch gekennzeichnet, dass**
- die zentrale Empfangseinheit (4) mit der Ein- /Ausgangssynchronisationseinheit (15) verbunden ist, welche mit einem Computer und dem Perturbationsmodul (100) verbunden ist, wobei während des gesamten Messvorgangs Signale vom Drucksensor und den Elektroden (3) mit Signalen vom mechanischen Perturbationsmodul (100) synchronisiert werden, und
- wobei die Signale dann an den Computer übertragen werden, der die Signale erfasst, speichert und anschließend verarbeitet, was ferner ein geeignetes Computerprogramm zur automatischen Analyse der erhaltenen Daten einschließt, um die Rumpfstabilität der Person zu bewerten.

4. Vorrichtung nach einem der vorstehenden Ansprüche, **dadurch gekennzeichnet, dass** die Vorrichtung ferner einen Sensor oder eine Elektrode oder eine andere Vorrichtung zur Verfolgung anderer Gesichtspunkte umfasst, die für die Stabilität des Rumpfes wichtig sind, wie eine Kraftmessplatte zur Verfolgung des Druckzentrums.

5. Vorrichtung nach einem der vorstehenden Ansprüche, **dadurch gekennzeichnet, dass**
- die Manschette (1) mindestens eine geschlossene aufblasbare Kammer aufweist, wobei die Vorrichtung bevorzugt als flexibler Gürtel oder als Manschette mit äußerem härterem Gehäuse aus Kunststoff oder Verbundmaterialien und einer inneren aufblasbaren Kammer aus flexiblem Material gefertigt ist;
- die Manschette (1) mit geeigneten Befestigungsmitteln (6) versehen ist, die es ermöglichen, die Manschette (1) um den Oberkörper einer Person zu legen und zuverlässig zu befestigen; und
- dass die Breite der Manschette (1) fünf bis dreißig Zentimeter, vorzugsweise etwa fünfzehn Zentimeter, betragen kann, sodass sie mindestens den Bereich zwischen der oberen Linie des Beckens (crista iliaca) und der untersten Linie des Brustkorbs abdeckt.

6. Vorrichtung nach dem vorstehenden Anspruch, **dadurch gekennzeichnet, dass** die aufblasbare Kammer eine erheblich verlängerte aufblasbare Kammer mit einer Öffnung ist, in die eine Vorrichtung für die Luftzufuhr oder Luftabfuhr eingebaut ist, wobei die Kammer aus Leinen, Kohlefasergewebe oder Kevlargewebe (Polyparaphenylenterephthalamid) hergestellt ist.

7. Vorrichtung nach einem der vorstehenden Ansprüche, **dadurch gekennzeichnet, dass**:
- an einer beliebigen Stelle innerhalb der Manschette (1) mindestens ein Drucksensor angebracht ist, oder, falls die Manschette (1) mehrere Fächer aufweist, jede der Kammern mindestens einen Drucksensor aufweist, wobei der Sensor aus leitfähigem Gummi, leitfähigem Textil, selbstklebenden Sensoren oder Metallsensoren unterschiedlicher Typen wie Ag-AgCI, Au, Cu bestehen kann; und
- dass die Muskelaktivität mit jeder geeigneten Elektrode (3) verfolgt werden kann, die in der Lage ist, die Muskelaktivität zu messen, wie EMG-Elektroden zur Messung der elektrischen Aktivität von Muskeln, insbesondere EMG-Textilelektroden und leitfähige Gummielektroden, wobei die Anzahl der Elektroden mindestens eine, vorzugsweise zwei oder mehr, am meisten bevorzugt vier beträgt.

8. Vorrichtung nach dem vorstehenden Anspruch, **dadurch gekennzeichnet, dass** die Elektroden (3) beweglich in/an einem geeigneten Halter (2) angebracht und/oder befestigt sind, so dass sie überall auf dem Gürtel positioniert werden können, um eine korrekte Position in Bezug auf die Körpermaße und -Form der Person zu gewährleisten, wobei die Elektroden vorzugsweise auf mindestens einem Muskel platziert werden müssen ausgewählt aus der Gruppe bestehend aus:
- m. obliques abdominis externus,
- m. obliques abdominis internus,
- m. rectus abdominis,
- m. errector spinae,
- m. multifidus;
vorzugsweise auf mehrere der genannten Muskeln oder einen zusätzlichen Bauch-/Lendenmuskel, der für die Rumpfstabilität sorgt.

9. Vorrichtung nach einem der vorstehenden Ansprüche, **dadurch gekennzeichnet, dass** das Perturbationsmodul (100) als Halter oder Stange (101) konzipiert ist, um in den Händen einer Person gehalten zu werden und mit einem Rückhalte-/Zugelement mit einem Seil oder einem Draht (12) verbunden ist, das in das Rückhalte-/Zugelement (105) hinein- oder herausgezogen werden kann; wobei somit das Perturbationsmodul zwei Betriebsarten ermöglicht:
- das Rückhalte-/Zugelement fungiert als Rückhaltevorrichtung, die konfiguriert ist, um eine gewisse Kraft auszuüben, die ein Ziehen des Seils oder des Drahtes (12) nach oben verhindert; wobei die Person die Stange (101) auf Schulterhöhe horizontal zum Boden ziehen und festhalten soll, wodurch die Rumpfmuskulatur aktiviert wird; und
- das Rückhalte-/Zugelement als Ziehvorrichtung fungiert, die konfiguriert ist, um das Seil oder den Draht (12) in das Element (105) hineinzuziehen, wobei die Person, welche die Stange hält, darauf abzielt, die Stange (101) in Höhe der Schultern horizontal zum Boden festzuhalten, was ebenfalls zur Aktivierung der Rumpfmuskulatur führt.

10. Vorrichtung nach einem der Ansprüche 1 bis 8, **dadurch gekennzeichnet, dass** das Perturbationsmodul (100) einen Beschleunigungsmesser zur Messung der Geschwindigkeitsänderung der Armbewegungen umfasst.

11. Vorrichtung nach einem der vorstehenden Ansprüche, **dadurch gekennzeichnet, dass** das Analysemodul die Eingangs-/Ausgangs-Synchronisationseinheit (15) umfasst, das mit dem Computer und dem Perturbationsmodul verbunden ist, wobei während des gesamten Messvorgangs die Signale des Drucksensors und der Elektroden (3) mit den Signalen des mechanischen Perturbationsmoduls (100) synchronisiert werden, und wobei die Signale vom Computer verarbeitet werden, um mindestens einige der folgenden Daten in dem Zeit- oder Amplitudenbereich zu erhalten:
- gegebenenfalls Muskelaktivität vor und nach dem Test;
- Muskelaktivität vor jeder Perturbation;
- Muskelaktivität während jeder Perturbation;
- Muskelaktivität nach jeder Perturbation;
- gegebenenfalls intraabdominaler Druck vor und nach dem Test;
- intraabdominaler Druck vor jeder Perturbation;
- intraabdominaler Druck während jeder Perturbation;
- intraabdominaler Druck nach jeder Perturbation;
- Dauer einer jeden Perturbation; und
- auf die Stange ausgeübte Kraft während jeder Perturbation.

12. Vorrichtung nach einem der vorstehenden Ansprüche, **dadurch gekennzeichnet, dass** die zentrale Empfangseinheit (4) und die Eingangs-/Ausgangs-Synchronisationseinheit (15) (a) Verstärker, die konfiguriert sind, um den Kraftsensor, Beschleunigungsmesser-, Druck- und EMG-Signale zu verstärken; (b) eine Analog-zu-Digital-Übertragungseinheit; (c) eine digitale Eingabe- /Ausgabeeinheit für die Steuerung der mechanischen Perturbationsauslösung; (d) eine Datenerfassungseinheit zum zeitsynchronen Auslesen der Signale durch einen Computer; und (e) eine kundenspezifische Computersoftware für die Speicherung, Visualisierung, Verarbeitung/Quantifizierung und Berichterstattung der erfassten Signale umfasst.

13. Verfahren zur Bewertung der Rumpfstabilität unter Verwendung der oben beschriebenen Vorrichtung nach den vorstehenden Ansprüchen, **dadurch gekennzeichnet, dass** das Verfahren mindestens die folgenden Schritte umfasst:
a) Anlegen der aufblasbaren Manschette (1) mit mindestens einer Elektrode (3) und mindestens einem Drucksensor um die Taille einer Person,
b) Herstellen des Kontakts zwischen der Person und dem Perturbationsmodul (100),
c) Auslösen mindestens einer Perturbation mit dem Perturbationsmodul (100),
d) Messen der Muskelaktivität und des Drucks innerhalb der Manschette (1),
e) Empfangen der Signale vom Sensor und der Elektrode (3) am zentralen Empfänger (4),
f) Übertragen von Signalen an die Eingangs-/Ausgangs-Synchronisationseinheit (15), die mit einem Computer und dem Perturbationsmodul (100) verbunden ist,
g) optionales Verarbeiten und Speichern der Signale im Computer, und
h) abschließendes Bewerten der Rumpfstabilität der Person auf der Grundlage der in den Schritten d) und optional g) erhaltenen Daten; alles im Zeit- oder Amplitudenbereich.

14. Verfahren nach dem vorstehenden Anspruch, **dadurch gekennzeichnet, dass** Perturbationen mindestens einmal, vorzugsweise mehr als einmal angewendet werden, um genauere Daten zu erhalten, wobei die bevorzugte Anzahl von Perturbationen 5 bis 20 beträgt, die zufällig in einem bestimmten Zeitraum verteilt sein können und unterschiedliche Kräfte aufweisen können.

15. Verfahren nach einem der Ansprüche 13 bis 14, **dadurch gekennzeichnet, dass** die inneren Perturbationen durch die Verwendung von Gewichten, z. B. bis zu 5 kg, verstärkt werden.

## Revendications

1. Un dispositif pour une évaluation non invasive de la stabilité fonctionnelle du tronc comprenant :
- un module sensitif, lequel module sensitif comprend :
∘ une sangle gonflable (1) qui doit être placée autour de la taille du sujet,
∘ au moins une électrode (3) montée sur ladite sangle (1) pour la mesure de l'activité musculaire des muscles lombaires et abdominaux,
∘ au moins un capteur de pression pour la mesure de la pression à l'intérieur de la sangle (1), correspondant à la pression intra-abdominale du sujet,
- un module d'analyse, une unité réceptrice centrale (4) pour la réception et la transmission des signaux provenant desdites électrodes (3) et dudit capteur de pression vers le module d'analyse,
**caractérisé en ce que**
- le dispositif comprend également un module de perturbation mécanique (100),
- où le module sensitif est synchronisé avec le module de perturbation mécanique (100), et
- où les électrodes (3) et le capteur sont configurés pour mesurer l'activité musculaire et la pression, respectivement, lors de l'application contrôlée de perturbations sur le tronc du sujet, causées par le module de perturbation.

2. Le dispositif selon la revendication 1, **caractérisé en ce que** le module de perturbation mécanique emploie des manœuvres de déstabilisation, à savoir :
- une perturbation externe, pour laquelle un mécanisme approprié est configuré afin de relâcher une tension prédéterminée.

3. Le dispositif selon l'une quelconque des revendications qui précèdent, **caractérisé en ce que**
- l'unité réceptrice centrale (4) est raccordée à l'unité de synchronisation entrée/sortie (15), qui est raccordée à un ordinateur et au module de perturbation (100), de façon à ce que durant tout le processus de mesure, les signaux provenant du capteur de pression et des électrodes (3) soient synchronisés avec les signaux provenant du module de perturbation mécanique (100), et
- de façon à ce que les signaux soient ensuite transmis à l'ordinateur, qui capte, stocke et par la suite traite les signaux, qui peut également impliquer un programme informatique approprié pour l'analyse automatique des données obtenues afin d'évaluer la stabilité du tronc du sujet.

4. Le dispositif selon l'une quelconque des revendications qui précèdent, **caractérisé en ce que** le dispositif comprend également tout capteur ou toute électrode ou tout autre appareil permettant de suivre d'autres aspects importants pour la stabilité du tronc, tels qu'une plateforme de force pour suivre le centre de pression.

5. Le dispositif selon l'une quelconque des revendications qui précèdent, **caractérisé en ce que**
- la sangle (1) possède au moins une chambre gonflable fermée, le modèle privilégié étant une ceinture flexible ou une sangle possédant un revêtement externe plus dur composé de plastiques ou de matériaux composites et d'une chambre interne gonflable faite en un matériau flexible ;
- la sangle (1) est fournie avec un système de fixation approprié (6), qui permet de positionner la sangle (1) autour du torse du sujet et de l'y fixer de façon fiable ; et
- la largeur de la sangle (1) peut être de cinq à trente centimètres, de préférence environ quinze centimètres, de sorte qu'elle couvre au moins la surface entre la limite supérieure du pelvis (crista iliaca) et la limite inférieure de la cage thoracique.

6. Le dispositif selon la revendication qui précède, **caractérisé en ce que** la chambre gonflable est une chambre fermée de forme considérablement allongée avec une ouverture, dans laquelle un appareil pour envoyer de l'air dans la chambre ou en retirer est installé, la chambre étant faite en toile, en tissu de fibres de carbone ou en Kevlar (poly-paraphénylène téréphthalamide).

7. Le dispositif selon l'une quelconque des revendications qui précèdent, **caractérisé en ce que** :
- au moins un capteur de pression est placé quelque part dans la sangle (1), ou dans le cas où la sangle (1) possède plus d'un compartiment, chacun des compartiments possède au moins un capteur de pression, ledit capteur pouvant être en caoutchouc conducteur, tissu conducteur, être un capteur auto-adhésif, un capteur métallique de différents types tels que Ag-AgCI, Au, Cu ; et
- l'activité musculaire est suivie par une électrode appropriée (3) capable de mesurer l'activité musculaire, telle que des électrodes d'EMG pour la mesure de l'activité électrique des muscles, en particulier des électrodes d'EMG en tissu et des électrodes en caoutchouc conducteur, le nombre desdites électrodes étant d'au moins un, de préférence deux ou plus, quatre étant encore préférable.

8. Le dispositif selon la revendication qui précède, **caractérisé en ce que** les électrodes (3) sont montées de manière mobile et/ou fixées dans/sur un support approprié (2), de façon à pouvoir être repositionnées n'importe où sur la ceinture afin de permettre une position correcte en fonction des mensurations et de la corpulence du sujet, les électrodes étant de préférence placées sur au moins un muscle sélectionné dans le groupe comprenant :
- m. obliques abdominis externus,
- m. obliques abdominis internus,
- m. rectus abdominis,
- m. errector spinae,
- m. multifidus ;
de préférence sur plusieurs desdits muscles ou tout autre muscle abdominal/lombaire impliqué dans la stabilité du tronc.

9. Le dispositif selon l'une quelconque des revendications qui précèdent, **caractérisé en ce que** le module de perturbation (100) est conçu comme un support ou une barre (101) que le sujet doit tenir dans les mains et qui est raccordé à un élément de retenue/traction comportant une corde ou un fil (12), qui peut rentrer ou sortir de l'élément de retenue/traction (105) ; le module de perturbation permettant ainsi deux modes d'utilisation :
- l'élément de retenue/traction fait office d'appareil de retenue, qui est configuré pour appliquer une certaine force qui empêche la traction de la corde ou du fil (12) vers le haut ; le sujet tenant la barre s'efforce de tirer et de tenir la barre (101) au niveau des épaules horizontalement par rapport aux sol, faisant fonctionner en conséquence les muscles du tronc ; et
- l'élément de retenue/traction fait office d'appareil de traction, qui est configuré pour tirer la corde ou le fil (12) dans l'élément (105), le sujet tenant la barre (101) s'efforçant de la maintenir au niveau des épaules à l'horizontale par rapport au sol, ce qui a également pour conséquence l'activation des muscles du tronc.

10. Le dispositif selon l'une quelconque des revendications 1 à 8, **caractérisé en ce que** le module de perturbation (100) comprend un accéléromètre pour mesurer le changement de vélocité du mouvement des bras.

11. Le dispositif selon l'une quelconque des revendications qui précèdent, **caractérisé en ce que** le module d'analyse comprend l'unité de synchronisation entrée/sortie (15), qui est raccordée à l'ordinateur et au module de perturbation, de façon à ce que durant tout le processus de mesure, les signaux provenant du capteur de pression et des électrodes (3) soient synchronisés avec les signaux provenant du module de perturbation mécanique (100), et de sorte que les signaux soient traités par l'ordinateur afin d'obtenir au moins certaines des données suivantes - dans le domaine temporel ou de l'amplitude :
- facultativement l'activité musculaire avant et après le test ;
- l'activité musculaire avant chaque perturbation ;
- l'activité musculaire pendant chaque perturbation ;
- l'activité musculaire après chaque perturbation ;
- facultativement la pression intra-abdominale avant et après le test ;
- la pression intra-abdominale avant chaque perturbation ;
- la pression intra-abdominale pendant chaque perturbation ;
- la pression intra-abdominale après chaque perturbation ;
- la durée de chaque perturbation ; et
- la force appliquée sur la barre pendant chaque perturbation.

12. Le dispositif selon l'une quelconque des revendications qui précèdent, **caractérisé en ce que** l'unité réceptrice centrale (4) et l'unité de synchronisation entrée/sortie (15) comprennent (a) des amplificateurs qui sont configurés pour amplifier les signaux du capteur de force, de l'accéléromètre, de pression et d'EMG ; (b) une unité de transmission analogique-vers-numérique ; (c) une unité d'entrée/sortie numériques pour le contrôle du déclenchement des perturbations mécaniques ; (d) une unité d'acquisition de données pour permettre à un ordinateur de lire les signaux de façon synchronisée dans le temps ; et (e) un logiciel informatique conçu sur mesure pour le stockage, la visualisation, le traitement/la quantification des signaux acquis, et la création de rapports sur ces derniers.

13. Une méthode pour l'évaluation de la stabilité du tronc, utilisant le dispositif décrit ci-dessus selon l'une quelconque des revendications qui précèdent, **caractérisée en ce que** la méthode comprend au moins les étapes suivantes :
a) le placement de la sangle gonflable (1) avec au moins une électrode (3) et au moins un capteur de pression autour de la taille d'un sujet,
b) l'établissement du contact entre le sujet et le module de perturbation (100),
c) le déclenchement d'au moins une perturbation par le biais du module de perturbation (100),
d) la mesure de l'activité musculaire et de la pression à l'intérieur de la sangle (1),
e) la réception des signaux provenant du capteur et de l'électrode (3) en direction du récepteur central (4),
f) la transmission des signaux à l'unité de synchronisation entrée/sortie (15), qui est raccordée à un ordinateur et au module de perturbation (100),
g) facultativement le traitement et le stockage des signaux dans l'ordinateur, et
h) l'évaluation finale de la stabilité du tronc du sujet sur la base des données obtenues lors des étapes d) et facultativement g) ; tout cela dans le domaine temporel ou de l'amplitude.

14. La méthode selon la revendication qui précède, **caractérisée en ce que** les perturbations sont appliquées au moins une fois, de préférence plus d'une fois afin d'obtenir des données plus précises, le nombre privilégié de perturbations étant de 5 à 20, qui peuvent être distribuées au hasard au cours d'un certain laps de temps et peuvent avoir différentes forces.

15. La méthode selon l'une quelconque des revendications 13 à 14, **caractérisée en ce que** les perturbations internes sont accrues par l'utilisation de poids, par exemple des poids allant jusqu'à 5 kg.
